# EUROPÄISCHE PATENTANMELDUNG

(11) **EP 4 837 952 A2**
(43) Veröffentlichungstag der Anmeldung: **11.11.2026**
(21) Anmeldenummer: 26164822.4
(22) Anmeldetag: 13.03.2026
(51) Int. Cl.: C12M 1/00, C12M 1/26, C12M 1/33, C05F 17/05

(54) **ANLAGE ZUR VERARBEITUNG VON ORGANISCHEM MATERIAL**

(30) Priorität: 14.03.2025 DE 202025101361 U
(71) Anmelder: MARMIX GmbH & Co. KG, 89597 Unterwachingen (DE); Biofa GmbH, 72525 Münsingen (DE); Kaesemann, Thomas, 87545 Burgberg (DE)
(72) Erfinder: Kaesemann, Thomas, 87545 Burgberg (DE)
(74) Vertreter: Kimpfbeck, Thomas

(57) **Zusammenfassung**

Es wird eine Anlage (100; 110; 130; 200) zur Verarbeitung von organischem Material mittels Insektenlarven vorgeschlagen. Diese Anlage (100; 110; 130; 200) umfasst eine Mischvorrichtung (112; 202) zum Mischen des organischen Materials; eine Insektenlarven-Dosiervorrichtung (215); eine Fördervorrichtung (220, 222) zum Fördern von wenigstens einem ersten Behälter (212), der mit gemischtem organischem Material und mit Insektenlarven befüllt ist. Die Anlage (100; 110; 130; 200) weist ferner ein erstes Modul (102; 202) auf, das die Mischvorrichtung (112; 202) umfasst, und ein zweites Modul (104; 118, 122; 214), das die Fördervorrichtung (220, 222) umfasst. Ferner werden ein modularer, mobiler Bioreaktor, eine Verwendung wenigstens eines standardisierten Containers, ein Sammelbehälter zum Sammeln von organischem Material, ein Transporter für organisches Material und ein System zur Verarbeitung von organischem Material vorgeschlagen.

## Beschreibung

### GEBIET DER ERFINDUNG

Die vorliegende Erfindung betrifft eine Anlage zur Verarbeitung von organischem Material, einen modularen, mobilen Bioreaktor, eine Verwendung wenigstens eines standardisierten Containers, einen Sammelbehälter zum Sammeln von organischem Material, einen Transporter für organisches Material und ein System zur Verarbeitung von organischem Material.

### STAND DER TECHNIK

Die Verwertung organischen Materials bzw. von Abfällen durch Insektenlarven, insbesondere durch Maden der Schwarzen Soldatenfliege (Hermetia illucens), hat in den letzten Jahren zunehmende Aufmerksamkeit erhalten. Solche Systeme bieten eine effiziente Möglichkeit zur Reduzierung organischer Reststoffe und zur Gewinnung wertvoller Biomasse in Form von Larvenprotein und -fett. Bekannte Verfahren setzen auf kontrollierte Bedingungen zur Optimierung der Zersetzungsleistung und des Wachstums der Larven.

Aus DE 102020002938 A1 ist ein Verfahren und eine Anordnung zum Recyceln organischer Abfälle bekannt. Das Verfahren besteht darin, die Abfälle Insektenlarven zuzuführen, welche diese als Nahrung verstoffwechseln. Endprodukte des Verfahrens sind Tierfutter und/oder Öls aus den Insektenlarven sowie Dünger aus den Ausscheidungen und nicht verstoffwechselter Abfälle. Vor dem Prozess werden die Abfälle mechanische auf eine Korngröße von unter 5 mm zerkleinert und unter Zugabe von Flüssigkeit zu einer Paste mit mehr als 50% Flüssigkeitsgehalt verarbeitet. Diese Paste wird zusammen mit den Insektenlarven in einen Behälter eingebracht. Nach dem Verstoffwechselungsprozess wird der Behälterinhalt in einer Trennstufe aufgetrennt. Die Insektenlarven werden ausgepresst, um Öls zu gewinnen, und anschließend als Tierfutter verwendet. Die Abfälle werden als Dünger ausgegeben.

Aus KR 20060030829 A ist eine Vorrichtung zur Behandlung von organischem Abfall unter Verwendung von Fliegenlarven bekannt. Herkömmliche Systeme zur Behandlung von Viehmist mit Fliegenlarven sind aufgrund ihrer hohen Kapazität und Kosten für kleine Viehzuchtbetriebe oft unwirtschaftlich. Zudem ist der Prozess umständlich, da Fliegeneier extern ausgebrütet und bei jeder Mistzugabe hinzugefügt werden müssen, was die Effizienz verringert und die Kosten erhöht. Die KR 20060030829 A löst dieses Problem durch den Einsatz von fünf intermittierend rotierenden Flachbandförderern innerhalb eines kompakten Behandlungstanks, der mit Moskitonetzen ausgestattet ist, um das Entweichen der Fliegen zu verhindern. Zusätzlich sind ein Heißluftgebläse und ein Ventilator installiert, um die Temperatur und die Feuchtigkeit zu regulieren. Am unteren Ende befindet sich eine Verpuppungsvorrichtung aus Stroh. Diese Lösung bietet eine einfache Handhabung, reduziert die Behandlungskosten und ist insbesondere für kleinere Betriebe geeignet.

Aus der EP 4108081 A1 ist eine multifunktionale modulare Einheit zur Züchtung von Insekten und zur Biobehandlung von Nebenprodukten und/oder organischen Abfällen und verwandte technologische Systeme zur elektromechanischen Lagerung und Handhabung bekannt.

Aus der RU2336696 C1 betrifft eine Anlage auf höhenverstellbaren Stützen. Die Anlage ist ausgelegt Larven mit Flüssigfutter zu füttern.

Aus der PL 41824416 A ist eine Anlage für die Aufzucht von flugunfähigen Insekten und/oder Larven bekannt. Diese bekannte Anlage hat ein autonomes Förderband mit seitlichen Wänden.

Aus der US 2017311612 A1 ist ein modulares Insektenproduktionssystem bekannt. Das Insektenproduktionssystem kann Module umfassen, die das Mischen von Rohstoffen, die verbesserte Aufteilung von Rohstoffen, das Füttern von Insekten, die Insektenzucht, das Sammeln von Insekten, das Zerkleinern von Insekten, das Entfernen von Krankheitserregern, das Mischen von multifunktionalem Mehl, das Mischen von Flüssigkeiten, das Formen, das Kochen, das Aromatisieren, das Mischen von Biokatalysatoren, das Trennen von Exoskeletten, das Trennen von Flüssigkeiten und die Extraktion von Lipiden umfassen. Das Insektenproduktionssystem kann so konfiguriert werden, dass er aus einer Vielzahl von Containermodulen aufgebaut ist.

Aus der WO 2023153948 A1 ist ein Container-Set zur Zucht und/oder Aufzucht von Insekten und/oder Larvenformen von Insekten und Verwendung des Container-Sets bekannt. Das bekannte Container-Set zur Zucht und/oder Aufzucht von Insekten und/oder Larvenformen von Insekten. Der Container enthält ein mehrstufiges Regal mit Zuchtflächen in Form von Regalen und/oder autonomen Förderbändern, die mit einem Futtertank verbunden sind, vorzugsweise über eine automatische Dosierpumpe. Der Container ist mit einem Stromversorgungssystem ausgestattet, das Beleuchtungslampen, ein Heizsystem sowie ein Lüftungs- und Klimatisierungssystem betreibt. Das Regal kann 1 bis 30 Stufen aufweisen, wobei die Container für den mobilen Einsatz mit einem Sattelanhänger verbunden werden können.

### AUFGABE DER ERFINDUNG

Somit besteht die Aufgabe der Erfindung darin eine Anlage zur Verarbeitung von organischem Material mittels Insektenlarven zu schaffen, die vollintegriert, transportabel, energieeffizient und modular ist.

### OFFENBARUNG DER ERFINDUNG

Erfindungsgemäß wird eine Anlage zur Verarbeitung von organischem Material mittels Insektenlarven gemäß Anspruch 1 zur Verfügung gestellt. Die erfindungsgemäße Anlage umfasst vorzugsweise eine Mischvorrichtung zum Mischen des organischen Materials; eine Insektenlarven-Dosiervorrichtung; eine Fördervorrichtung zum Fördern von wenigstens einem ersten Behälter, der mit gemischtem organischem Material und mit Insektenlarven befüllt ist; ein erstes Modul, das die Mischvorrichtung umfasst und ein zweites Modul, das die Fördervorrichtung umfasst.

Organisches Material ist im Wesentlichen ein Substrat oder ein Material, auf oder in dem ein Organismus leben kann. Das organische Material kann ein heterogenes, festes Ausgangsmaterial, wie z.B. pflanzliche und/oder tierische Stoffe, Biomüll, Abfallprodukte aus der Lebensmittelherstellung, Grünschnitt, Gärreste aus Biogasanlagen, Molkerei- und Schlachtabfall, Reststoffe aus Fischmehl und Eipulverherstellung, kontaminierte Substrate aus oder mit organischen Komponenten, Klärschlamm, Trester, Zuckerrübenschnitzel, Algen, Melasse und/oder Leinöl. Das organische Material dient vorzugsweise als Futter für die Insektenlarven.

Als Insektenlarven kommen vorzugsweise Junglarven zum Einsatz. Junglarven sind insbesondere Larven, die bereits aus einem Insektenei geschlüpft sind. Die Insektenlarven reifen z.B. über eine Zeit von 10 bis 14 Tagen zu erwachsenen Larven heran, während sie das organische Material fressen. Ein Eiweißgehalt der Insektenlarven kann sich dabei bis zu einem Schwellwert erhöhen. Anders gesagt, in dem wenigstens ersten Behälter kann sich eine Insektenlarve bzw. Junglarve bis zum Erwachsenenstadium entwickeln. Ist der Eiweißschwellwert erreicht kann hochwertiges Eiweiß bzw. Protein geerntet werden, wobei das organische Material weitgehend verarbeitet bzw. entsorgt wurde. Das Protein kann z.B. als Fisch- oder allgemein, als Tierfutter verwendet werden.

Zu den Larven zählen allgemein Maden und Raupen. Für die vorliegende Erfindung eignen sich z.B. Maden, insbesondere die Larven bzw. Junglarven von Waffenfliegen. Noch geeigneter sind die Larven der schwarzen Soldatenfliege, auch Hermetia Illucens genannt. Hermetia Illucens kann typischerweise bis zu einem Drittel des Futtergewichts in Körpermasse umwandeln. Hermetia Illucens enthält dabei ca. 58% Protein.

Die Mischvorrichtung dient insbesondere dazu das organische Material zu homogenisieren, damit die Insektenlarven das Substrat gleichmäßiger und schneller verarbeiten können. Die Mischvorrichtung umfasst zum Beispiel einen Rührlöffel oder einen Schneckenmischer. Die Mischvorrichtung kann eine integrierte Zerkleinerungsvorrichtung umfassen, zum Beispiel eine Schneide und/oder eine Reißkante. Eine integrierte Misch- und Zerkleinerungsvorrichtung dient insbesondere zum effizienten Zerkleinern und Homogenisieren des Substrats. Die Mischvorrichtung kann einen Ein oder Mehrschneckenmischer umfassen. Ferner kann die Mischvorrichtung vertikal oder horizontal in dem wannenartigen Behälter angeordnet sein, d.h. einen Vertikal- bzw. Horizontalmischer bilden. Ferner kann die Mischvorrichtung einen Paddelmischer und/oder Freifall-Paddelmischer umfassen.

Die Mischvorrichtung kann einen wannenartigen Behälter umfassen, der insbesondere nach oben geöffnet ist. Die Öffnung kann zum Beschicken des wannenartigen Behälters mit organischem Material dienen. Der wannenartige Behälter umfasst insbesondere mindestens vier Seitenwände und einen Boden. Das Material des Behälters umfasst im Wesentlichen Metall, insbesondere Stahlblech. Ein oder mehrere der Seitenwände können mit Rippen verstärkt sein. Ein oder mehrere Seitenwände und/oder der Boden können insbesondere doppelwandig und/oder mit Dämmmaterial ausgestattet sein. Am oberen Rand einer der Seitenwände ist insbesondere der öffenbare Deckel mit einem Scharnier angelenkt. Der Deckel kann zum Beschicken des wannenartigen Behälters geöffnet werden. Der Deckel kann einen hydraulischen oder elektrischen Aktor zum Öffnen und Schließen umfassen. Der Deckel kann thermisch isoliert sein.

Gemäß einer weiteren Ausgestaltung kann die Fördervorrichtung eine Vorschubeinrichtung umfassen, die den ersten Behälter entlang der Fördervorrichtung fördert. Die Vorschubeinrichtung kann eine motorgetriebene Förderkette oder einen motorgetriebenen Spindelvortrieb umfassen. Die Vorschubeinrichtung kann den ersten Behälter entlang des zweiten Moduls zu fördern. Die Anlage kann eine Computersteuerung aufweisen. Die Computersteuerung kann eine Fördergeschwindigkeit der Vorschubeinrichtung in Abhängigkeit der Verarbeitungsgeschwindigkeit des organischen Materials durch die Insektenlarven einstellen. Die Vorschubeinrichtung kann den ersten Behälter kontinuierlich oder schubweise fördern. Die Anlagen kann wenigstens einen verfahrbaren Regalwagen aufweisen, den ersten und/oder weitere Behälter umfassen kann, wobei der Regalwagen insbesondere mittels der Fördervorrichtung gefördert wird. Der Regalwagen kann Fahrrollen aufweisen und mit der Fördervorrichtung abnehmbar in Eingriff gebracht werden. Der Regalwagen kann mehrere Schienen oder Regalböden aufweisen, die insbesondere die Behälter aufnehmen.

In einer weiteren Ausführung kann die Anlage ein drittes Modul aufweisen, wobei das zweite Modul und das dritte Modul die Fördervorrichtung umfassen. Die Anlage kann alternativ vier, fünf, zehn oder bis zu n Module umfassen, wobei eine n eine positive, ganze Zahl ist. Die Module 2 bis n können die Fördervorrichtung umfassen. Die Anzahl der Module kann in Abhängigkeit von verschiedenen Kriterien gewählt werden, z.B. Anzahl der Behälter, Durchsatzes von organischem Material, Verarbeitungsgeschwindigkeit der Insektenlarven und/oder Platzverhältnisse an einem Aufstellort für die erfindungsgemäße Anlage.

Gemäß einer Weiterbildung kann ein erster standardisierter Container das erste Modul umfassen und ein zweiter standardisierter Containerkann das zweite Modul umfassen. In anderen Worten ein standardisierter Containerkann ein Modul bilden. Ein standardisierter Container umfasst vorzugsweise einen ISO-Container bzw. genormten Großraumbehälter aus Stahl. Standardisierte Containersind leicht, etwa per Lastkraftwagen, transportierbar und ermöglichen die Anlage modular auszulegen.

In einer weiteren Ausgestaltung kann das erste und/oder zweite Modul eine Logistikeinrichtung, insbesondere eine Einrichtung zum Verladen, Befördern, Lagern und/oder Entlagern, umfassen. Die Logistikeinrichtung kann eine am Frachtcontainer angeordnete Kranöse, Stapelvorrichtung und/oder Befestigungsvorrichtung umfassen.

Gemäß einer Weiterbildung kann die Anlage eine Schleuseneinrichtung umfassen. Die Schleuseneinrichtung kann Modul bzw. einen Frachtcontainer umfassen. Die Schleuseneinrichtung dient vorzugsweise zum dichten bzw. hermetischen weiterleiten von Behältern oder Regalwagen von einem Modul zu einem weiteren Modul bzw. von einem Frachtcontainer zu einem weiteren Frachtcontainer. Die Anlage kann mehrere Frachtcontainer bzw. Module umfassen, die in einer Reihe am Boden angeordnet sind. Ferner kann die Anlage mehrere mäander- oder Schlangenförmig angeordnete Module umfassen. Außerdem kann die Anlage mehrere vertikal übereinander angeordnete Module umfassen. Um die Behälter bzw. Regalwagen von einem Modul zum nächsten Modul zu fördern kann eine dazwischen angeordnete Schleuseneinrichtung als Ventil, als Aufzugsvorrichtung und/oder als Richtungsänderungsvorrichtung wirken.

Die Verarbeitung von organischem Material kann unangenehme oder belastende Gerüche hervorrufen, die Anlage ist vorzugsweise dicht bzw. hermetisch ausgelegt und kann eine Desodorierungsvorrichtung umfassen. Gemäß einer weiteren Ausgestaltung kann die Anlage, insbesondere die Mischvorrichtung, einen Hygienisator umfassen. Der Hygienisator kann das organische Material hygienisieren bzw. pasteurisieren. Das organische Material kann pathogene Erreger, wie Viren, Bakterien und Pilze enthalten. Zur Hygienisierung des organischen Materials ist im Allgemeinen eine über einen bestimmten Zeitraum konstant gehaltene und hohe Temperatur erforderlich. Beispielsweise kann eine Erhitzung des organischen Materials auf über 70°C für die Dauer von einer Stunde erfolgen. Eine Temperatur von über 70°C während der Dauer von einer Stunde kann zum Beispiel Madenfrass bzw. Insektenkot hygienisieren.

Die Anlage kann eine Trennungsvorrichtung oder Siebvorrichtung umfassen, um die fertig gemästeten Maden von Reststoffen zu trennen. Die Anlage kann eine Blanchiervorrichtung umfassen, die die fertig gemästeten Maden mittels mind. 70° heißem Wasser blanchiert. Die Anlage kann ein Mahlwerk zum Zerkleinern der gemästeten Maden umfassen.

Der Begriff hygienisieren ist im Allgemeinen definiert als die Behandlung von Bioabfällen mit dem Ziel Mikroorganismen mit pathogener Wirkung auf Kulturpflanzen, Tiere und Menschen so weit abzutöten, dass nur noch ein geringes Risiko einer Übertragung von Krankheiten besteht.

Gemäß einer weiteren Ausgestaltung kann die Anlage einen Fluidkanal zur Wärmerückgewinnung umfassen. Der erste und/oder zweite standardisierte Container kann den Fluidkanal umfassen, wobei der Fluidkanal in einer Außenhülle des ersten und/oder zweiten standardisierten Containers angeordnet ist. Der Fluidkanal kann einen zweiten Boden, eine Zwischendecke oder eine zweite Wand in einem standardisierten Container bzw. einem Modul bilden. Die Insektenlarven können beim Heranwachsen, Verarbeiten bzw. Fressen des organischen Materials Wärme erzeugen. Zur Kühlung bzw. Temperaturregulierung kann Wärme von einem n-ten Modul über den Fluidkanal abtransportiert und am Anfang der Anlage zur Vorwärmung der Junglarven wieder abgegeben werden. Dabei kann ein Wärmetauscher, Ventilator und/oder Ventil eingesetzt werden. Die Rückgewinnung von Wärme und Verwendung derselben am Anfang der Anlage zum Vorwärmen von Junglarven kann zu einer besseren Energieeffizient der Anlage führen. Vorzugsweise weisen alle Module oder alle standardisierten Container bzw. alle Modul 2 bis n bzw. standardisierte Container2 bis n einen doppelten Boden, doppelte Wand oder doppelte Decke zur Wärmerückführung auf.

In einer weiteren Ausgestaltung kann die Mischvorrichtung einen ersten Schneckenmischer, einen zweiten Schneckenmischer, einen dritten Schneckenmischer, eine Zerkleinerungsvorrichtung, ein Temperaturmessinstrument, ein Feuchtemessinstrument, pH-Wertmessinstrument, eine Wägeeinrichtung, eine Belüftungsvorrichtung, eine Befeuchtungsvorrichtung, einen Wärmetauscher und/oder eine Heizung umfassen. Die Schneckenmischer dienen vorzugsweise zum homogenen Mischen des organischen Materials. Die Zerkleinerungsvorrichtung dient vorzugsweise zum Zerkleinern des organischen Materials. Die Instrumente, die Wägeeinrichtung, die Belüftungsvorrichtung, die Befeuchtungsvorrichtung, der Wärmetauscher und/oder die Heizung können mit der Computersteuerung zusammenwirken, um die Verarbeitung des organischen Materials in der Anlage zu steuern oder zu überwachen.

Gemäß einer Weiterbildung kann die Anlage, insbesondere das erste und/oder zweite Modul, eine Pumpe zum Befüllen des wenigstens ersten Behälters mit dem gemischten organischen Material umfasst. Die Pumpe kann eine Perestaltikpumpe umfassen. Eine Perestaltikpumpe ist vorzugsweise ein geschlossenes System. Das kann den Vorteil bilden, dass Material in einem Schlauch der Perestaltikpumpe eingeschlossen bleibt. Perestaltikpumpen fördern schonend, sind einfach im Aufbau und kostengünstig im Unterhalt.

Gemäß einer weiteren Ausgestaltung kann die Anlage ein Viskosimeter umfassen. Das Viskosimeter kann die Viskosität des gemischten organischen Materials ermitteln. Ist das organische Material zu flüssig, so können die Maden ertrinken, ist es zu fest so können die Maden nicht in die Tiefe des Materials vordringen. Die Perestaltikpumpe wird vorzugsweise in Abhängigkeit einer ermittelten Viskosität des gemischten organischen Materials gesteuert.

In einer weiteren Ausgestaltung kann die Anlage, insbesondere die Insektenlarven-Dosiervorrichtung, eine pneumatische Fördervorrichtung, insbesondere einen Luftstromförderer, umfassen. Die Insektenlarven sind im Allgemeinen sehr empfindlich auf mechanische Beanspruchung. Der Luftstromförderer kann die Insektenlarven schonend und umgeben von polsternder Luft zu den Behältern fördern und auf dem gemischten organischen Material ablegen.

Außerdem wird ein modularer, mobiler Bioreaktor zur Verarbeitung von organischem Material zu Frass und zur Madenzucht vorgeschlagen. Der Bioreaktor kann umfassen: einen ersten standardisierten Container, der einen Mischer zum Mischen und Zerkleinern des organischen Materials zu Madenfutter, einen Hygienisator zum Hygienisieren des organischen Materials und eine Madenfutterpumpe umfasst; einen Maden-Luftstromförderer; mehrere Wagen, die jeweils mehrere Behälter umfassen, wobei die Madenfutterpumpe die Behälter mit Madenfutter beschickt und die Maden-Luftstromförderer das Madenfutter in den Behältern mit Maden der Soldatenfliege beschickt; einen zweiten standardisierten Containermit einer Wagenvorschubvorrichtung zum Fördern der Wagen entlang des zweiten standardisierten Containers;
einem dritten standardisierten Containermit einer weiteren Wagenvorschubvorrichtung zum Fördern der Wagen entlang des dritten standardisierten Containers; einen Abwärmeluftkanal, der in einer Außenhülle des zweiten und dritten standardisierten Containers angeordnet ist, um Abwärmeluft zum ersten standardisierten Container zu führen.

Ferner wird eine Verwendung wenigstens eines standardisierten Containers vorgeschlagen, um einen modularen Madenzuchtreaktor herzustellen. Ein Verfahren zur Herstellung von Madenfrass mit der erfindungsgemäßen Anlage oder dem erfindungsgemäßen Bioreaktor wird vorgeschlagen.

Des Weiteren wird ein Sammelbehälter zum Sammeln von organischem Material mit einer Einwurföffnung, einem fluiddichten Behälter und einem Entladeverschluss vorgeschlagen, wobei die Einwurföffnung eine fluiddichte Schleuse umfasst. Organisches Material, z.B. Speisereste oder organischem Abfall, kann belastende Gerüche hervorrufen. Gerade in subtropischen und tropischen Gegenden ist das Sammeln von organischem Abfall schwierig. Der erfindungsgemäße Sammelbehälter ist vorzugsweise fluiddicht ausgelegt. Fluiddicht umfasst flüssigkeitsdicht, gasdicht, hermetisch dicht, geruchsdicht und/oder druckdicht. Der Sammelbehälter kann in Wohnanlagen, bei Restaurants und dergleichen aufgestellt werden. Die Einwurföffnung kann als Zellenradschleuse ausgelegt sein. Die Zellenradschleuse ist ein Mechanismus, der ein dosiertes Einspeisen von organischem Material in den fluiddichten Behälter ermöglicht, wobei wenig oder gar keine Gerüche oder Gase aus dem fluiddichten Behälter entweichen. Die Zellenradschleuse weist vorzugsweise wenigstens zwei gegenüberliegende Einwurf-Sektoren auf, die ein Einspeisen von zwei Seiten des Sammelbehälters ermöglichen. Der Sammelbehälter weist vorzugsweise eine mechanische Betätigungsvorrichtung, etwa ein Fußpedal, auf, um die Zellenradschleuse zu drehen. Beim Drehen der Zellenradschleuse werden die Einwurf-Sektoren über Öffnungen des fluiddichten Behälters gedreht, sodass das organische Material durch Schwerkraft in das Innere des fluiddichten Behälters gelangt. Der Sammelbehälter kann ein Überdruckventil und eine Desodorierungsvorrichtung, insbesondere mit einer Gaskartusche, umfassen. Entsteht in dem Sammelbehälter durch biologische Prozesse Gase, so können diese bei Überschreiten eines vorbestimmten Drucks durch das Überdruckventil abgelassen und mittels der Desodorierungsvorrichtung desodoriert werden.

Gemäß einer weiteren Ausgestaltung umfasst der Sammelbehälter vorzugsweise eine Reinigungsvorrichtung. Die Reinigungsvorrichtung kann wenigstens eine Waschdüse und/oder eine Vibrationsvorrichtung umfassen. Die Reinigungsvorrichtung kann einen Wasseranschluss umfassen, der mit einem Transportfahrzeug verbindbar ist. Haftet organisches Material an einer Innenwand des fluiddichten Behälters an, so kann die Reinigungsvorrichtung helfen das organische Material von der Innenwand zu lösen. Die wenigstens eine Waschdüse kann eine Druckbegrenzung aufweisen, die dazu dient, den Sammelbehälter in Vibration zu versetzen, um anhaftendes organische Material zu lösen.

Gemäß einer weiteren Ausführung wird ein Transporter vorgeschlagen, der ein Nutzfahrzeug, einen Kran und einen Tank zur Aufnahme und Speicherung des organischen Materials umfassen kann, wobei der Tank vorzugsweise eine Misch- und/oder Zerkleinerungsvorrichtung umfasst.

Des Weiteren wird ein System zur Verarbeitung von organischem Material vorgeschlagen, das wenigstens einen erfindungsgemäßen Sammelbehälter, einen erfindungsgemäßen Transporter und die erfindungsgemäße Anlage umfasst. Der Kran kann den Sammelbehälter anheben und über dem Tank anordnen. Wird der Entladeverschluss geöffnet, so kann der Inhalt des Sammelbehälters in den Tank entleert werden. Der Transporter kann zu verschiedenen Sammelbehältern fahren und deren Inhalte aufsammeln. Während der Fahrt kann der Transporter das gesammelte organische Material zerkleinern und mischen. Am Ende der Fahrt kann der Transporter das gemischte und zerkleinerte Material der erfindungsgemäßen Anlage zuführen. Vorzugsweise kann das bereits zerkleinerte und gemischte organische Material direkt in den wenigstens ersten Behälter gepumpt werden.

### KURZBESCHREIBUNG DER ZEICHNUNGEN

Ausführungsbeispiele der Erfindung werden anhand der Zeichnungen und der nachfolgenden Beschreibung näher erläutert. Es zeigen:
- Figur 1A: eine Draufsicht auf ein Ausführungsbeispiel einer Anlage gemäß der Erfindung.
- Figur 1B: eine Draufsicht auf ein weiteres Ausführungsbeispiel einer Anlage gemäß der Erfindung,
- Figur 1C: eine Seitenansicht auf ein weiteres Ausführungsbeispiel einer Anlage gemäß der Erfindung,
- Figur 2: ein weiteres Ausführungsbeispiel einer Anlage gemäß der Erfindung
- Figur 3: ein Ausführungsbeispiel eines Sammelbehälters gemäß der Erfindung und
- Figur 4: ein Ausführungsbeispiel eines Transporters gemäß der Erfindung.

### AUSFÜHRUNGSFORMEN DER ERFINDUNG

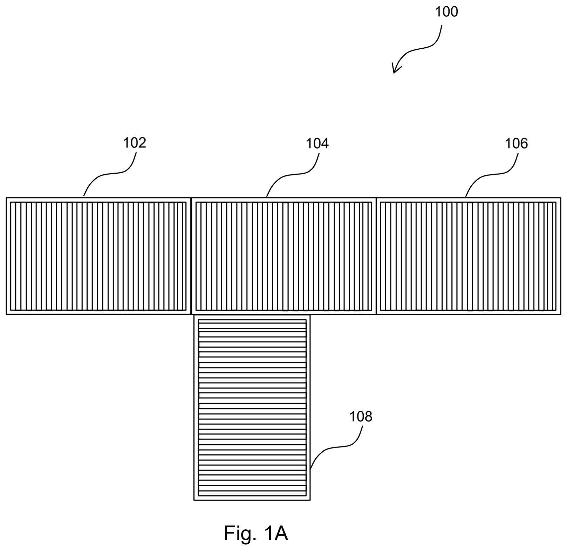

In der Figur 1A zeigt eine Draufsicht auf eine Anlage 100 zur Verarbeitung von organischem Material gezeigt. Die Anlage 100 umfasst mehrere standardisierte Container 102, 104, 106, 108, die Module der Anlage 100 umfassen. Der standardisierte Container 102 umfasst eine Mischvorrichtung zum Mischen des organischen Materials. Die standardisierten Container104 und 106 umfassen je eine Fördervorrichtung zum Fördern von mehreren Wagen, die mit Behältern versehen sind. Gemisches organisches Material wird mit einer Perestaltikpumpe von der Mischvorrichtung zu den Behältern gepumpt, wenn diese im standardisierten Container104 verortet sind. Eine Luftstrompumpe fördert Junglarven der schwarzen Soldatenfliege zu den mit gemischtem organischem Material gefüllten Behältern. Die Fördervorrichtungen fördern den Wagen mit gefüllten Behältern entlang der standardisierten Container104 und 106. Der standardisierte Container108 umfasst eine Schleuseneinrichtung, die ausgelegt ist mit Wagen mit leeren Behältern beschickt zu werden. Die Schleuseneinrichtung hat zwei Tore, die abwechselnd öffenbar sind. Die Anlage 100 kann weitere standardisierte Containeraufweisen, in denen die Wagen mit gefüllten Behältern so lange entlang gefördert werden, bis die Larven fertig gemästet bzw. adult sind und einen bestimmten Eiweißgehalt erreicht haben. Die Wagen können am Ende der Anlage über eine weitere Schleuseneinrichtung ausgeschleust werden.

Die Anlage 100 ist durch den Einsatz von standardisierten Containern modular und transportabel. Jeder standardisierte Containerkann für eine eigene Aufgabe in der Verarbeitung des organischen Materials ausgelegt werden, sodass alle nötigen Verarbeitungsschritte in der Anlage voll integriert werden können.

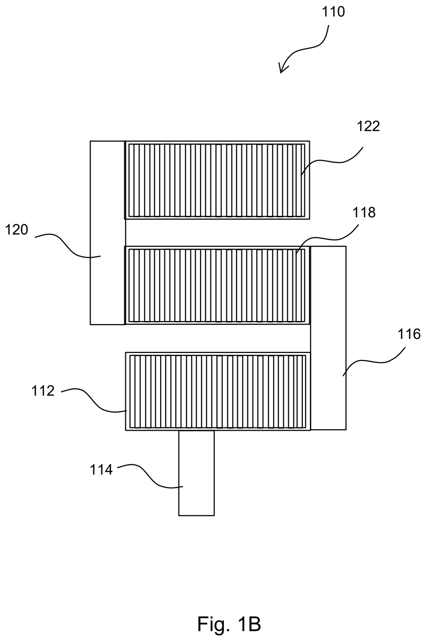

In der Figur 1B ist eine Draufsicht auf eine Anlage 110 gezeigt. Die Anlage 110 umfasst ein Mischmodul 112, die einer Mischvorrichtung entspricht, ein Einschleusemodul 114, ein Transportschleusemodul 116, ein Prozessmodul 118, ein weiteres Transportschleusemodul 120 und ein weiteres Prozessmodul 122. Die Module mäanderförmig angeordnet, um eine kleine Stellfläche optimal auszunutzen. Das Einschleusemodul 114, das Transportschleusemodul 116 und das weiteres Transportschleusemodul 120 entsprechen einer Schleuseneinrichtung.

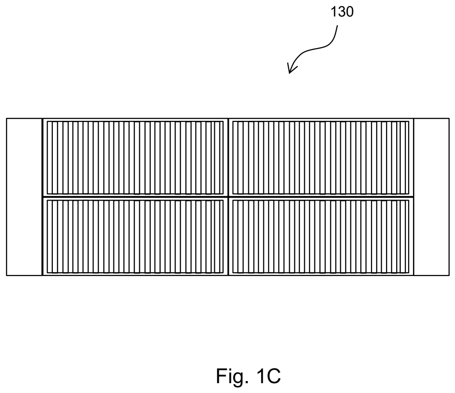

In der Figur 1C ist eine Seitenansicht einer Anlage 130 gezeigt. Die Anlage 130 weist mehrere vertikal übereinander gestapelte und nebeneinander angeordnete standardisierte Container auf. Die Anlage 130 ist dadurch sehr platzsparend.

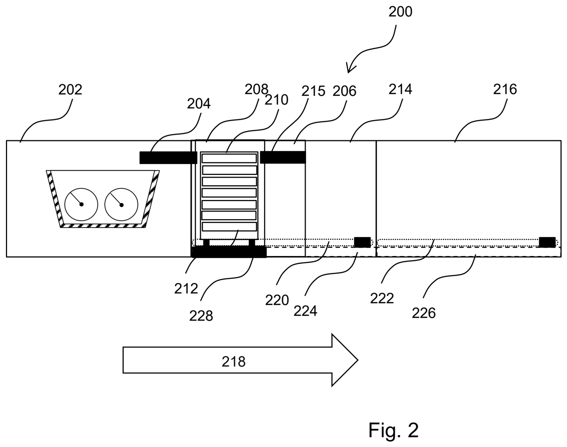

In der Figur 2 ist eine Anlage 200 gezeigt. Die Anlage 200 umfasst ein Schneckenmischer-Modul 202 zum Mischen und Zerkleinern von Lebensmittelabfällen, eine Perestaltikpumpe 204 zum Pumpen der gemischten und zerkleinerten Lebensmittelabfälle. Ferner umfasst die Anlage 200 eine Schleuse 206 mit einem äußeren Tor 208 und einem inneren Tor (nicht gezeigt), die abwechselnd öffnen. Durch die Schleuse 206 wird ein Wagen 210 mit mehreren leeren Behältern 212 in die Anlage eingeschleust. Die Anlage 200 umfasst prozessabwärts, vgl. Pfeil 218, zwei Madenzuchtmodule 214 und 216 sowie weitere Madenzuchtmodule (nicht gezeigt). Im Madenzuchtmodul 214 ist eine Luftstromförderanlagen 215 angeordnet. Die Luftstromförderanlage 215 fördert Insektenlarven zu den leeren Behältern 212.

Die zwei Madenzuchtmodule 214, 216 umfassen jeweils eine Förderkette 220, 222 zum Fördern des Wagens entlang des Prozessablaufs 218 sowie Wärmeluftstromkanäle in doppelten Böden 224 und 226. Prozessaufwärts bzw. am Anfang der Anlage ist das Schneckenmisch-Modul 202 und die Schleuse 206 angeordnet. Aufgrund der Erhöhung der doppelten Böden 224, 226 ist zum Einfahren des Wagens eine Rampe 228 erforderlich, die vor dem äußeren Tor 208 angeordnet ist

Die Anlage 200 ist modular aufgebaut und kann zerlegt leicht transportiert bzw. nach Kundenwünschen mit mehr oder weniger Modulen konfiguriert werden. Jedes Modul kann für eine eigene Aufgabe im Prozess ausgelegt werden, sodass die Anlage alle nötigen Verarbeitungsschritte voll integriert. Die Wärmeluftstromkanäle in den doppelten Böden 224, 226 ermöglichen die Rückführung von Abwärme am Prozessende zum Prozessanfang, sodass die Energieeffizienz der Anlage gesteigert werden kann.

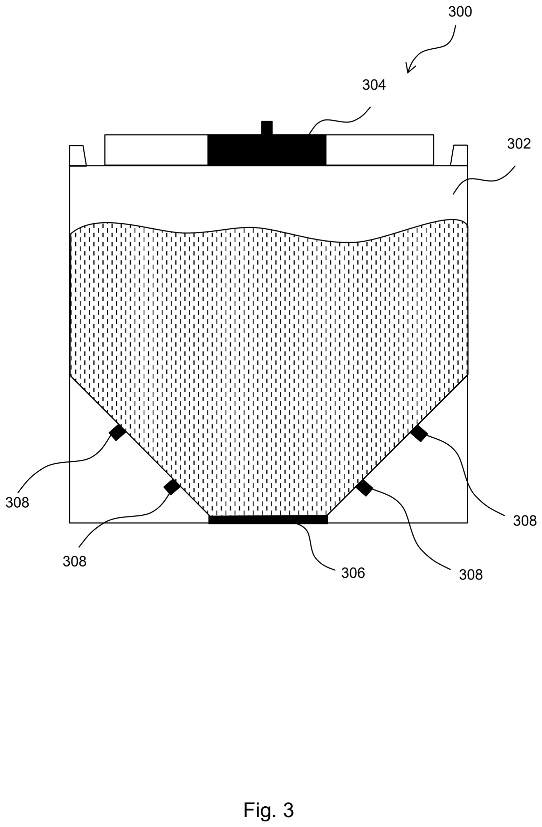

In der Figur 3 ist ein Sammelbehälter 300 gezeigt. Der Sammelbehälter 300 weist einen gasdichten Behälter 302, Zellenradschleuse 304 und einen Entladeverschluss 306 auf. Die Zellenradschleuse 304 ist um eine vertikale Achse drehbar auf einer Oberseite des gasdichten Behälters 302 angeordnet. Der gasdichte Behälter 302 ist zylinderförmig und nach unten siloartig verjüngend zum Entladeverschluss 306 hin ausgelegt. Organisches Material, z.B. Speisereste oder organischem Abfall, kann belastende Gerüche hervorrufen. Gerade in subtropischen und tropischen Gegenden ist das Sammeln von organischem Abfall schwierig. Der Sammelbehälter 300 ist gasdicht ausgelegt, um diese Gerüche zu verringern oder zu vermeiden. Der Sammelbehälter 300 kann in Wohnanlagen, bei Restaurants und dergleichen aufgestellt sein. Die Zellenradschleuse 304 erlaubt ein dosiertes Einspeisen von organischem Material in den gasdichten Behälter 302, auf diese Weise kann verhindert werden, dass zu große, den Sammelbehälter evtl. verstopfende, Gegenstände eingefüllt werden. Die Zellenradschleuse 304 weist zwei gegenüberliegende Einwurf-Sektoren auf, die ein Einspeisen von zwei Seiten des Sammelbehälters ermöglichen. Der Sammelbehälter 300 weist ein Fußpedal (nicht gezeigt) auf, um die Zellenradschleuse zu drehen. Beim Drehen der Zellenradschleuse werden die Einwurf-Sektoren über Öffnungen des gasdichten Behälters 302 gedreht, sodass das organische Material durch Schwerkraft in das Innere des gasdichten Behälters 302 gelangt. Der Sammelbehälter weist ein Überdruckventil und eine Desodorierungsvorrichtung auf. Entsteht in dem Sammelbehälter 300 durch biologische Prozesse Gase, so können diese bei Überschreiten eines vorbestimmten Drucks durch das Überdruckventil abgelassen und mittels der Desodorierungsvorrichtung desodoriert werden. Der Sammelbehälter 300 umfasst mehrere Waschdüsen 308. Die Waschdüsen 308 sind mit einem Wasseranschluss des Sammelbehälters 300 verbunden, der mit einem Transportfahrzeug verbindbar ist. Haftet organisches Material an einer Innenwand des gasdichten Behälters 302 an, so helfen die Wasserdüsen das organische Material von der Innenwand zu lösen. Die Waschdüse 308 können eine Druckbegrenzung aufweisen, die dazu dient, den Sammelbehälter 300 in Vibration zu versetzen, um anhaftendes organische Material zu lösen.

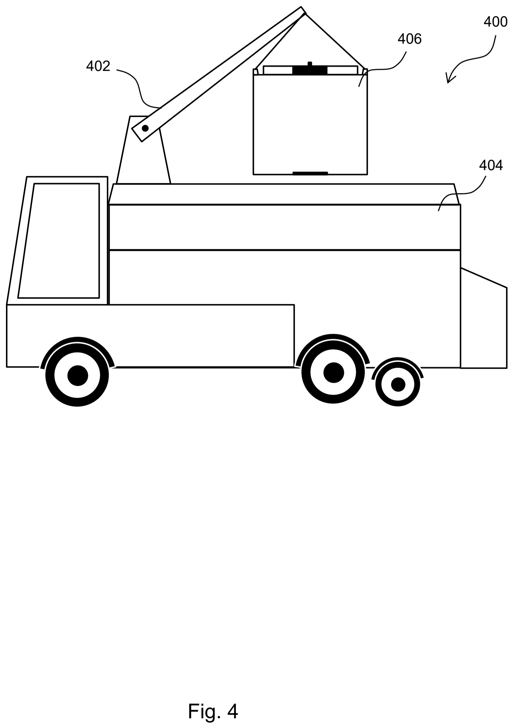

In der Figur 4 ist ein Transporter 400 mit Ladekran 402 und einem Tank 404. Der Tank 404 weist zwei Schneckenmischer (nicht gezeigt) auf. Der Ladekran 402 hebt einen Sammelbehälter 406 für organisches Material an. Wird das organische Material in den Tank 404 entladen, so können die zwei Schneckenmischer das organische Material während der Fahrt zerkleinern und mischen.

### BEZUGSZEICHENLISTE

- 100: Anlage
- 102: standardisierter Container
- 104: standardisierter Container
- 106: standardisierter Container
- 108: standardisierter Container

- 110: Anlage
- 112: Mischmodul
- 114: Einschleusemodul
- 116: Transportschleusemodul
- 118: Prozessmodul
- 120: weiteres Transportschleusemodul
- 122: weiteres Prozessmodul

- 130: Anlage

- 200: Anlage
- 202: Schneckenmischer-Modul
- 204: Perestaltikpumpe
- 206: Schleuse
- 208: äußeres Tor
- 210: Wagen
- 212: Behälter
- 214: Madenzuchtmodul
- 215: Luftstromförderanlage
- 216: Madenzuchtmodul
- 218: Prozessablauf
- 220: Förderkette
- 222: Förderkette
- 224: doppelter Boden
- 226: doppelter Boden
- 228: Rampe

- 300: Sammelbehälter
- 302: gasdichter Behälter
- 304: Zellenradschleuse
- 306: Entladeverschluss
- 308: Waschdüsen

- 400: Transporter
- 402: Ladekran
- 404: Tank
- 406: Sammelbehälter

## Patentansprüche

1. Anlage (100; 110; 130; 200) zur Verarbeitung von organischem Material mittels Insektenlarven mit:
einer Mischvorrichtung (112; 202) zum Mischen des organischen Materials;
einer Insektenlarven-Dosiervorrichtung (215);
einer Fördervorrichtung (220, 222) zum Fördern von wenigstens einem ersten Behälter (212), der mit gemischtem organischen Material und mit Insektenlarven befüllt ist
**dadurch gekennzeichnet, dass**
die Anlage (100; 110; 130; 200) eine erstes Modul (102; 202) aufweist, das die Mischvorrichtung (112; 202) umfasst und dass die Anlagen ein zweites Modul (104; 118, 122; 214) aufweist, das die Fördervorrichtung (220, 222) umfasst.

2. Anlage (100; 110; 130; 200) zur Verarbeitung von organischem Material mittels Insektenlarven nach Anspruch 1
**dadurch gekennzeichnet, dass**
die Fördervorrichtung (220, 222) eine Vorschubeinrichtung (220, 222) umfasst, die den ersten Behälter (212) entlang der Fördervorrichtung (220, 222) fördert.

3. Anlage (100; 110; 130; 200) zur Verarbeitung von organischem Material mittels Insektenlarven nach Anspruch 1 oder 2
**dadurch gekennzeichnet, dass**
die Anlage (100; 110; 130; 200) ein drittes Modul (106; 216) aufweist, wobei das zweite Modul (104, 214) und das dritte Modul (106; 216) die Fördervorrichtung (220, 222) umfassen.

4. Anlage (100; 110; 130; 200) zur Verarbeitung von organischem Material mittels Insektenlarven nach einem der vorhergehenden Ansprüche
**dadurch gekennzeichnet, dass**
ein erster standardisierter Container (102) das erste Modul (102; 202) umfasst und ein zweiter standardisierter Container (104) das zweite Modul (104; 118, 122; 214) umfasst.

5. Anlage (100; 110; 130; 200) zur Verarbeitung von organischem Material mittels Insektenlarven nach einem der vorhergehenden Ansprüche
**dadurch gekennzeichnet, dass**
das erste (102; 202) und/oder zweite Modul (104; 118, 122; 214) eine Logistikeinrichtung, insbesondere eine Einrichtung zum Verladen, Befördern, Lagern und/oder Entlagern, umfassen.

6. Anlage (100; 110; 130; 200) zur Verarbeitung von organischem Material mittels Insektenlarven nach einem der vorhergehenden Ansprüche
**dadurch gekennzeichnet, dass**
die Anlage (100; 110; 130; 200) eine Schleuseneinrichtung (114, 116, 120; 206) umfasst.

7. Anlage (100; 110; 130; 200) zur Verarbeitung von organischem Material mittels Insektenlarven nach einem der vorhergehenden Ansprüche
**dadurch gekennzeichnet, dass**
die Anlage (100; 110; 130; 200) wenigstens einen verfahrbaren Regalwagen (210) aufweist, der den ersten Behälter (212) umfasst, wobei die Fördereinrichtung (220, 222) den Regalwagen (210) fördert.

8. Anlage (100; 110; 130; 200) zur Verarbeitung von organischem Material mittels Insektenlarven nach einem der vorhergehenden Ansprüche
**dadurch gekennzeichnet, dass**
die Anlage (100; 110; 130; 200), insbesondere die Mischvorrichtung (112; 202), einen Hygienisator umfasst.

9. Anlage (100; 110; 130; 200) zur Verarbeitung von organischem Material mittels Insektenlarven nach einem der vorhergehenden Ansprüche
**dadurch gekennzeichnet, dass**
die Anlage (100; 110; 130; 200) einen Fluidkanal (224, 226) zur Wärmerückgewinnung umfasst.

10. Anlage (100; 110; 130; 200) zur Verarbeitung von organischem Material mittels Insektenlarven nach Anspruch 9
**dadurch gekennzeichnet, dass**
der erste (102) und/oder zweite standardisierte Container (104) den Fluidkanal umfasst, wobei der Fluidkanal in einer Außenhülle des ersten und/oder zweiten standardisierten Containers angeordnet ist.

11. Anlage (100; 110; 130; 200) zur Verarbeitung von organischem Material mittels Insektenlarven nach einem der vorhergehenden Ansprüche
**dadurch gekennzeichnet, dass**
die Mischvorrichtung (112; 202) einen ersten Schneckenmischer, einen zweiten Schneckenmischer, einen dritten Schneckenmischer, eine Zerkleinerungsvorrichtung, ein Temperaturmessinstrument, ein Feuchtemessinstrument, pH-Wertmessinstrument, eine Wägeeinrichtung, eine Belüftungsvorrichtung, eine Befeuchtungsvorrichtung, einen Wärmetauscher und/oder eine Heizung umfasst.

12. Anlage (100; 110; 130; 200) zur Verarbeitung von organischem Material mittels Insektenlarven nach einem der vorhergehenden Ansprüche
**dadurch gekennzeichnet, dass**
die Anlage (100; 110; 130; 200), insbesondere das erste (102; 202) und/oder zweite Modul (104; 214), eine Pumpe (204) zum Beschicken des wenigstens ersten Behälters (212) mit dem gemischten organischen Material umfasst.

13. Anlage (100; 110; 130; 200) zur Verarbeitung von organischem Material mittels Insektenlarven nach Anspruch 12
**dadurch gekennzeichnet, dass**
die Pumpe (204) eine Perestaltikpumpe (204) umfasst.

14. Anlage (100; 110; 130; 200) zur Verarbeitung von organischem Material mittels Insektenlarven nach einem der vorhergehenden Ansprüche
**dadurch gekennzeichnet, dass**
die Anlage ein Viskosimeter umfasst.

15. Anlage (100; 110; 130; 200) zur Verarbeitung von organischem Material mittels Insektenlarven nach einem der vorhergehenden Ansprüche
**dadurch gekennzeichnet, dass**
die Anlage (100; 110; 130; 200), insbesondere die Insektenlarven-Dosiervorrichtung (215), eine pneumatische Fördervorrichtung (215), insbesondere einen Luftstromförderer (215), umfasst.

16. Modularer, mobiler Bioreaktor (100; 110; 130; 200) zur Verarbeitung von organischem Material zu Frass und zur Madenzucht mit:
einem ersten standardisierten Container (102), der einen Mischer (112; 202) zum Mischen und Zerkleinern des organische Material zu Madenfutter, einen Hygienisator zum Hygienisieren des organischen Materials und eine Madenfutterpumpe (204) umfasst;
einem Maden-Luftstromförderer (215);
mehreren Wagen (210), die jeweils mehrere Behälter (212) umfassen, wobei die Madenfutterpumpe (204) die Behälter (212) mit Madenfutter beschickt und die Maden-Luftstromförderer (215) das Madenfutter in den Behältern (212) mit Maden der Soldatenfliege beschickt;
einem zweiten standardisierten Container (104) mit einer Wagenvorschubvorrichtung (220, 222) zum Fördern der Wagen (210) entlang des zweiten standardisierten Containers (104);
einem dritten standardisierten Container (106) mit einer weiteren Wagenvorschubvorrichtung (222) zum Fördern der Wagen entlang des dritten standardisierten Containers (106);
einem Abwärmeluftkanal (224, 226), der in einer Außenhülle des zweiten (104) und dritten standardisierter Containers (106) angeordnet ist, um Abwärmeluft zum ersten standardisierten Container (104) zu führen.

17. Verwendung wenigstens eines standardisierten Containers, um einen modularen Madenzuchtreaktor herzustellen.

18. Sammelbehälter (300) zum Sammeln von organischem Material mit
einer Einwurföffnung (304),
einem fluiddichten Behälter (302) und
einem Entladeverschluss (306)
**dadurch gekennzeichnet, dass**
die Einwurföffnung (304) eine fluiddichte Schleuse (304) umfasst

19. Sammelbehälter (300) nach Anspruch 18
**dadurch gekennzeichnet, dass**
die Einwurföffnung (304) eine Zellenradschleuse (304) umfasst.

20. Sammelbehälter (300) nach Anspruch 18 oder 19
**dadurch gekennzeichnet, dass**
der Sammelbehälter (300) eine Reinigungsvorrichtung (308) umfasst.

21. Sammelbehälter (300) nach Anspruch 19
**dadurch gekennzeichnet, dass**
die Reinigungsvorrichtung (308) wenigstens eine Waschdüse (308) und/oder Vibrationsvorrichtung (308) umfasst.

22. Transporter (400) für organisches Material mit
einem Nutzfahrzeug (400),
einem Kran (402),
einem Tank (404) zur Aufnahme und Speicherung des organischen Materials,
**dadurch gekennzeichnet, dass**
der Tank (404) eine Misch- und/oder Zerkleinerungsvorrichtung umfasst.

23. System zur Verarbeitung von organischem Material umfassend:
wenigstens einen Sammelbehälter (300) nach einem der Ansprüche 18 bis 21,
einen Transporter (400) nach Anspruch 22 und
eine Anlage (100; 110; 130; 200) nach einem der Ansprüche 1 bis 15.
